# EUROPEAN PATENT APPLICATION

(11) **EP 2 005 909 A2**
(43) Date of publication of application: **24.12.2008**
(21) Application number: 07740641.1
(22) Date of filing: 30.03.2007
(51) Int. Cl.: A61B 17/58, A61F 2/28, A61F 2/44, A61L 27/00

(54) **DEVICE FOR VERTEBRAL REPAIR**

(30) Priority: 05.04.2006 JP 2006104265
(71) Applicant: Olympus Terumo Biomaterials Corp., Shinjuku-ku Tokyo 163-0914 (JP)
(72) Inventor: OJIMA, Satoshi, Nishitokyo-shi Tokyo 202-0002 (JP)
(74) Representative: von Hellfeld, Axel
(86) International application number: PCT/JP2007/057205
(87) International publication number: WO 2007/116833

(57) **Abstract**

The present invention enables, with a simple structure and without increasing the diameter of the device, a space inside a vertebral bone to be filled with a filler material while preventing leakage through cracks formed in the vertebral bone by compression fractures or the like, thereby reducing the length of time required for treatment. The present invention provides a device for vertebral repair used in procedures to repair vertebral fractures, comprising a pipe-like tubular body that is inserted into an introduction hole formed in a vertebral arch pedicle and can fill the vertebral bone interior with a filler material through an internal through-hole, and a sheet member attached in a folded state to the front end of the tubular body so as to obstruct the opening at the front end, wherein the sheet member is attached in such a manner as to separate from the tubular body when subjected to a compressive force from the filler material introduced through the through-hole in the tubular body.

## Description

### Technical Field

The present invention relates to a device for vertebral repair, for use in procedures to repair vertebral fractures.

### Background Art

Conventional examples of this type of a device are disclosed in patent citations 1 and 2.
In the device disclosed in Patent Citation 1, an inflatable tube is secured to the end of a catheter tube capable of insertion into an introduction hole formed in a pedicle of a vertebral arch. With this device, the deflated tube is introduced into the space inside the vertebral bone and inflated by injecting a substance such as normal saline solution through the catheter tube and into the tube, thereby spreading the cancellous bone and other matter inside the vertebral bone to make room for the filler material to be introduced later.

In the device disclosed in Patent Citation 2, an inflatable mesh bag is attached to the end of a catheter tube capable of insertion into an introduction hole formed in a pedicle of a vertebral arch. With this device, the inflatable bag is introduced into the space inside the vertebral bone in a deflated state, and inflated by an infusion of filler material via the catheter tube, and finally the opening to the inflatable bag is closed off.
Patent Citation 1: Japanese Translation of PCT International Application, Publication No. 2002-516697
Patent Citation 2: Japanese Translation of PCT International Application, Publication No. 2004-534612

### Disclosure of Invention

However, a potential problem with the device of Patent Citation 1 is that as the device serves merely to secure space for the filler material, if cracks are formed in the vertebral bone due to a compression fracture or other condition, the injected filler material may leak out through these cracks.
The device of Patent Citation 2 does not share this problem with Patent Citation 1 because the inflatable bag containing the filler material is left in the space inside the vertebral bone. However, with the device of Patent Citation 2, closing off the opening to the inflatable mesh bag can present difficulties.

One disadvantage is that the structure for closing off the fill opening inside the vertebral bone is unavoidably complex, and because this necessitates a larger diameter for the front end portion of the device, introducing the device smoothly into the introduction hole provided in the vertebral bone is difficult. Furthermore, the task of closing the fill opening inside the vertebral body from outside the body of the patient is difficult, and requires a considerably lengthy treatment.

The present invention has been developed in light of the above circumstances, and has an object of providing a device for vertebral repair that enables, with a simple structure and without increasing the diameter of the device, a space inside a vertebral bone to be filled with a filler material while preventing leakage through cracks formed in the vertebral bone by compression fractures or the like, thereby reducing the length of time required for treatment.

The present invention provides the following aspects to achieve this object.
The present invention is a device for vertebral repair used in procedures to repair vertebral fractures, comprising a pipe-like tubular body that is inserted into an introduction hole formed in the vertebral bone and can fill the vertebral bone interior with a filler material through an internal through-hole, and a sheet member attached in a folded state to the front end of the tubular body so as to obstruct the opening at the front end, wherein the sheet member is attached in such a manner as to separate from the tubular body when subjected to a compressive force from the filler material introduced through the through-hole in the tubular body.

According to the present invention, when the tubular body with the sheet member attached to the front end thereof is introduced into an introduction hole formed in a vertebral bone, preferably a vertebral arch pedicle, and a filler material is introduced through the through-hole in the tubular body, the compressive force from the filler material causes the sheet member to separate from the tubular body, release into the space inside the vertebral bone, and undergo expansion. Attaching the sheet member in such a manner as to separate from the tubular body when subjected to a compressive force from the filler material eliminates the need for a large-scale release mechanism, and prevents the front end part of the device from increasing in size. Furthermore, because the sheet member is attached so as to obstruct the opening at the front end of the tubular body, the sheet member acts as a barrier to the filler material discharged from the opening, and the filler material is prevented from progressing beyond the sheet member. As a result, any cracks present in the vertebral bone are blocked by the expanded sheet material, thereby preventing the filler material from leaking through the cracks.

In the invention described above, the sheet member may be folded along the outer surface of the front end of the tubular body, so as to expand under a compressive force from the filler material introduced through the through-hole in the tubular body.
By employing such a construction, the diameter of the device can be minimized during insertion through the introduction hole by providing the sheet member along the outer surface of the front end of the tubular body, and once the device is inserted into the vertebral bone, the sheet member can be separated from the tubular body and expanded by introducing the filler material. By using the compressive force applied by the filler material as the force to expand the sheet member, the need for a special power source or expansion mechanism is eliminated, allowing the device for vertebral repair to have a simple construction.

Furthermore, in the invention described above, the sheet member may be folded along the outer surface of the front end of the tubular body, and made of a material that expands to a remembered shape when inserted into the vertebral bone.
By employing such a construction, the diameter of the device can be minimized during insertion through the introduction hole by providing the sheet member along the outer surface of the tubular body, and once the device is inserted into the vertebral bone, the sheet member can be separated from the tubular body and expanded by introducing the filler material. By constructing the sheet member from a material such as a shape memory alloy or shape memory resin that expands to a predetermined shape in response to temperature or other conditions, the need for a special power source or expansion mechanism is eliminated, allowing the device for vertebral repair to have a simple construction.

Furthermore, the invention described above may comprise an outer sheath that is insertable into the introduction hole and is provided such that the portion of the sheet member folded along the outer surface of the front end of the tubular body is sandwiched between the outer sheath and the tubular body.
By employing such a construction, the folded sheet member can be sandwiched between the tubular body and the sheath member during insertion through the introduction hole formed in the vertebral arch pedicle, and issues such as the sheet member adhering to the inside wall of the introduction hole, or inadvertently separating or expanding during the insertion process, can be more reliably prevented. The release and expansion of the sheet member can be performed easily by removing the outer sheath after the sheet member has entered the space within the vertebral bone. Because the only function required of the outer sheath is to keep the sheet member in a folded state, the outer sheath need not be particularly thick, which prevents the front end portion of the device from increasing in diameter.

Furthermore, in the invention described above, the sheet member may be made of a flexible biocompatible material such as polylactic acid or tetrafluoroethylene.
Moreover, in the invention described above, the sheet member may be formed as a mesh that is finer than the particle diameter of the filler material.
This enables cells and body fluids such as blood to infiltrate the filler material through the holes in the mesh, thereby promoting the regeneration of biological tissue in the filler material. On the other hand, by using a mesh that is finer than the particle diameter of the filler material, the filler material can be prevented from leaking through the holes in the mesh.

Furthermore, in the invention described above, the opening at the front end of the tubular body may be provided so that the filler material that is provided via the through-hole in the tubular body is discharged in directions transverse to the longitudinal direction of the tubular body.
When the sheet member expands due to the compressive force from the filler material, if the opening faces the longitudinal direction of the tubular body and there is too little resistance in the forward discharge direction of the filler material, the sheet member may be forced in the discharge direction without having the opportunity to fully expand. With this aspect of the present invention, because the filler material is discharged in a direction transverse to the longitudinal direction of the tubular body, the compressive force of the filler material also acts strongly in a direction that serves to expand the sheet member, allowing the sheet member to be expanded more reliably even when resistance is low.

The present invention demonstrates an effect whereby with a simple structure and without increasing the diameter of the device, a space inside a vertebral bone can be filled with a filler material while preventing leakage through cracks in the vertebral bone caused by compression fractures or the like, allowing the time required for treatment to be reduced.

### Brief Description of Drawings

[FIG. 1] A perspective view showing a device for vertebral repair according to an embodiment of the present invention.
[FIG. 2] A drawing describing the use of the device for vertebral repair of FIG. 1 in a procedure to repair a vertebral fracture.
[FIG. 3] A drawing showing the insertion of the device for vertebral repair according to this embodiment into an introduction hole provided in the vertebral bone, in the procedure to repair a vertebral fracture of FIG. 2.
[FIG. 4] A longitudinal sectional view explaining the process by which a sheet member is separated from a tubular body by the application of a compressive force to a filler material.
[FIG. 5] A longitudinal sectional view explaining the process by which the sheet member is expanded by injecting a filler material through the opening at the front end of the tubular body.
[FIG. 6] A longitudinal sectional view showing a modification of the device for vertebral repair of FIG. 1.
[FIG. 7] A longitudinal sectional view explaining the process by which the sheet member is separated from the tubular body in the device for vertebral repair of FIG. 6.
[FIG. 8] A longitudinal section showing another modification of the device for vertebral repair of FIG. 1.
[FIG. 9] A longitudinal section showing yet another modification of the device for vertebral repair of FIG. 1.

### Explanation of Reference:

- 1:: Device for vertebral repair
- 2:: Tubular body
- 2a:: Front end opening
- 2c:: Through-hole
- 3:: Sheet member
- 4:: Vertebral arch pedicle
- 5:: Introduction hole
- 6:: Filler material
- 7:: Vertebral bone
- 9:: Outer sheath

### Best Mode for Carrying Out the Invention

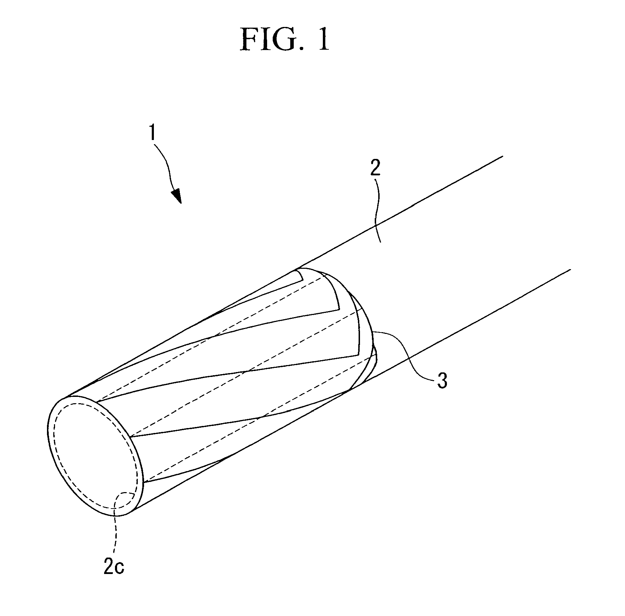

A device for vertebral repair 1 according to an embodiment of the present invention is described below with reference to FIG. 1 through FIG. 5.
As shown in FIG. 1, the device for vertebral repair 1 according to the present embodiment comprises a circular pipe-like tubular body 2, and a sheet member 3 attached to the end of the tubular body 2.

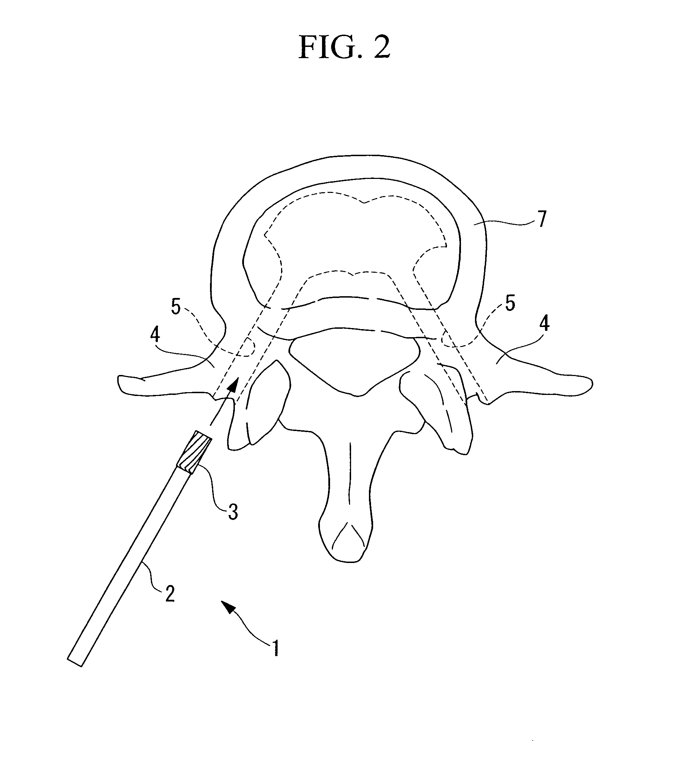

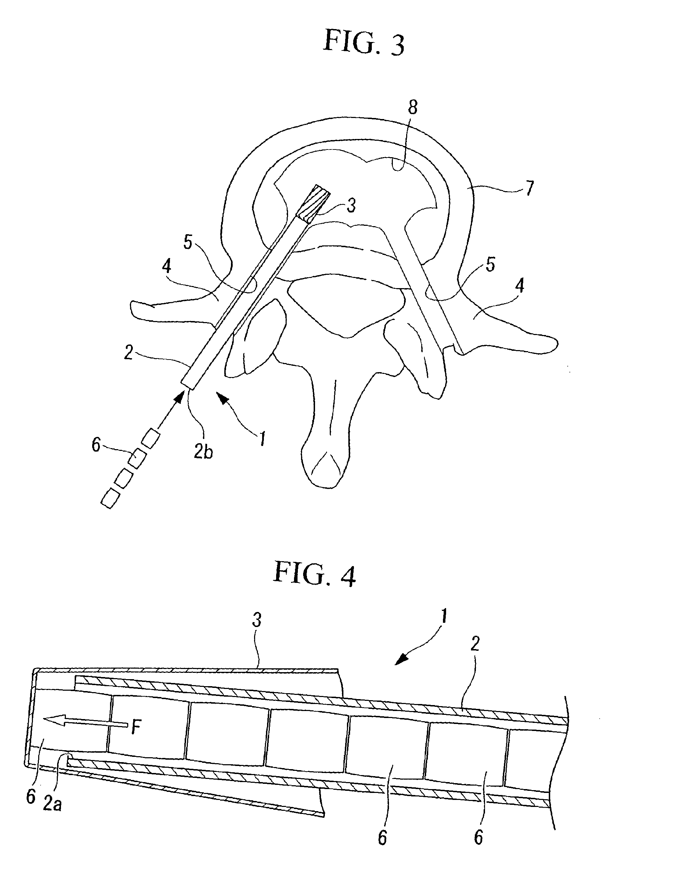

As shown in FIG. 2 and FIG. 3, the outer dimensions of the tubular body 2 permit insertion into an introduction hole 5 formed in a vertebral arch pedicle 4. Furthermore, the tubular body 2 comprises a through-hole 2c, the inner dimensions of which permit the introduction of a filler material 6 such as a bioactive cement or a sintered body of calcium phosphate such as a granular or particulate hydroxyapatite.

The sheet member 3 is, for example, formed from a flexible thin film of a material such as polylactic acid. By folding the sheet member 3 along the outer surface of the tubular body 2, the sheet member 3 forms a cylinder that is closed at one end. In cylindrical form, the sheet member 3 has an inner diameter dimension that is slightly larger than the outer diameter dimension of the tubular body 2. Thus, inserting one end of the tubular body 2 into the sheet member 3 gives a configuration in which the sheet member 3 is disposed along the outer surface of the tubular body 2 while also covering a front end opening 2a of the tubular body 2.

In order to maintain the shape of the folded sheet member 3, the edge of the sheet member 3 may be adhered to the outer surface of the tubular body 2 by an adhesive or the like.
This adhesion need only be strong enough to maintain the shape of the sheet member, and can preferably be released easily. Rather than relying on adhesion, the tubular body 2 can simply be inserted into the folded sheet member 3, provided that this configuration allows the shape of the sheet member 3 to be maintained to some extent.

A procedure to repair a vertebral fracture using such a device for vertebral repair 1 according to the present embodiment is described below.
As shown in FIG. 2, in order to perform a vertebral fracture repair procedure using the device for vertebral repair 1 according to the present embodiment, an introduction hole 5 is formed in the vertebral arch pedicle 4, and a cavity 8 is formed by curetting the inside of a vertebral bone 7. Next, the end of the device for vertebral repair 1 to which the sheet member 3 is attached is inserted into the introduction hole 5 from outside the vertebral bone 7.

Because the sheet member 3 is attached to the tubular body 2 so as to block the front end opening 2a thereof, by maneuvering the tubular body 2 through the introduction hole 5, the sheet member 3 is guided towards the cavity 8 in the vertebral bone 7. Because the sheet member 3 is folded along the outer surface of the tubular body 2, the outer dimensions of the front end of the device for vertebral repair 1 are only moderately increased, and the device remains easy to insert. Accordingly, the introduction hole 5 need not have a large diameter, and smooth insertion is possible even through an introduction hole 5 that is relatively small in diameter.

Furthermore, because the sheet member 3 is adhered in a folded state to the outer surface of the tubular body 2, the sheet member 3 maintains a cylindrical shape during the insertion process. Even in cases where adhesion is not employed, when the sheet member 3 is inside the introduction hole 5, the introduction hole 5 prevents the sheet member 3 from opening, and thus the sheet member 3 remains in cylindrical form throughout the insertion process.

Moreover, as shown in FIG. 3, by positioning the front end of the tubular body 2 inside the cavity 8 in the vertebral bone 7, the sheet member 3, which is attached to the front end of the tubular body 2, is positioned inside the cavity 8 in the vertebral bone 7. In this state, the filler material 6 is introduced from a rear opening 2b of the tubular body 2. If the filler material 6 is a fluid, the fluid can be introduced by applying pressure from a pump or other means, whereas if the filler material 6 is a solid in granular or particulate form, the solid can be introduced by applying pressure from a plunger or other device (not shown in the figure).

In this case, with the device for vertebral repair 1 of the present embodiment, because the front end opening 2a of the tubular body 2 is blocked by the sheet member 3, the introduced filler material 6 applies pressure to the sheet member 3 at the front end opening 2a. The sheet member 3, being only simply adhered to the tubular body 2 or attached only by folding, is easily separated from the tubular body 2 by the compressive force F applied by the filler material 6, as shown in FIG. 4.

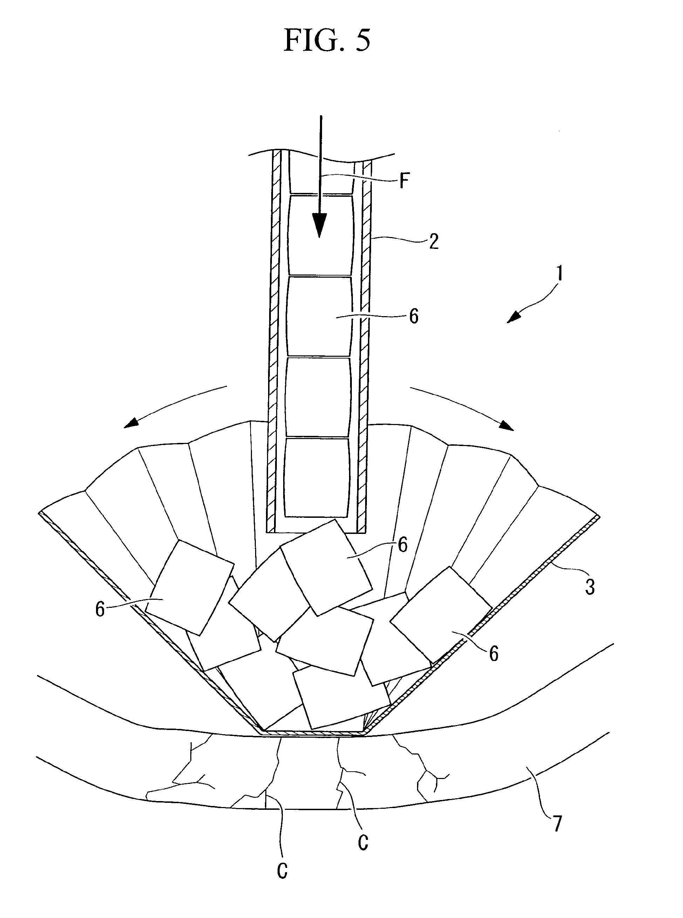

Furthermore, with continuous application of the compressive force F to the filler material 6, the filler material 6 discharged from the front end opening 2a of the tubular body 2 accumulates inside the sheet member 3 which is folded into a cylindrical shape, and as shown in FIG. 5, the sheet member 3 gradually unfurls in a manner that extends the folds therein. As a result, the sheet member 3, which was folded into a cylindrical shape, expands into a flat shape.

In this case, with the present embodiment, because the sheet member 3 is attached so as to block the front end opening 2a of the tubular body 2, the sheet member 3 in its expanded state is located in front of the tubular body 2 in the direction in which the filler material 6 is discharged from the front end opening 2a, thus serving to prevent the filler material 6 from progressing beyond the sheet member 3.

In other words, compression fractures of the vertebrae often manifest as cracks in the ventral wall of the vertebral bone 7, and when the filler material 6 is introduced through the introduction hole 5 provided in the vertebral arch pedicle 4, the filler material 6 travels towards the cracks C in the wall surface of the vertebral bone 7. With the device for vertebral repair 1 according to the present embodiment, because the sheet member 3 has expanded in the space between the front end opening 2a of the tubular body 2 and the fractured wall surface prior to the introduction of the filler material 6, the sheet member 3 prevents the filler material 6 discharged from the front end opening 2a of the tubular body 2 from leaking out through the cracks C.

Moreover, with continued supply of the filler material 6, the sheet member 3 is pushed against the wall surface containing the cracks C by the filler material 6, which further enhances the sealing effect on the cracks C. As a result, the filler material 6 can increase the pressure inside the vertebral bone 7, thereby expanding and elevating the vertebral bone 7. The fracture of the vertebral bone 7 is thereby repaired.

In this manner, with the device for vertebral repair 1 according to the present embodiment, simply by introducing the filler material 6, the resulting compressive force causes the sheet member 3 to separate from the tubular body 2, which removes the requirement for a special separation mechanism, prevents the front end part of the device for vertebral repair 1 from increasing in diameter, and simplifies the construction of the device. Furthermore, there is no concern that the separation process will fail, and the vertebral bone 7 can be elevated by a simple maneuver.

Furthermore, with the device for vertebral repair 1 according to the present embodiment, expansion of the sheet member 3 can be performed by the compressive force of the filler material 6 alone, eliminating the need for a special power source or expansion mechanism. Accordingly, the diameter of the front end part of the device for vertebral repair 1 can be further decreased, and the construction of the device further simplified.

Furthermore, leakage of the filler material 6 can be prevented by simply extending the folds of the sheet member 3 until a flat shape is formed, and there is no need to insist upon strict encapsulation of the filler material 6. Accordingly, there is no need to close off the filling hole in the sheet member 3 after the filler material 6 is introduced, which simplifies the procedure for repairing vertebral fractures, and reduces the length of time required for treatment.

An example was presented above in which a flexible thin film was used as the sheet member 3 in the device for vertebral repair 1 according to the present embodiment, but when the filler material 6 is a solid, such as a sintered body of a granular or particulate calcium phosphate, the sheet member may be a mesh that is finer than the particle diameter of the filler material 6. By employing such a construction, body fluids such as blood and marrow are able to pass through the holes in the mesh into the area containing the filler material 6, allowing the filler material 6 to serve as the foundation for the regeneration of biological tissue.

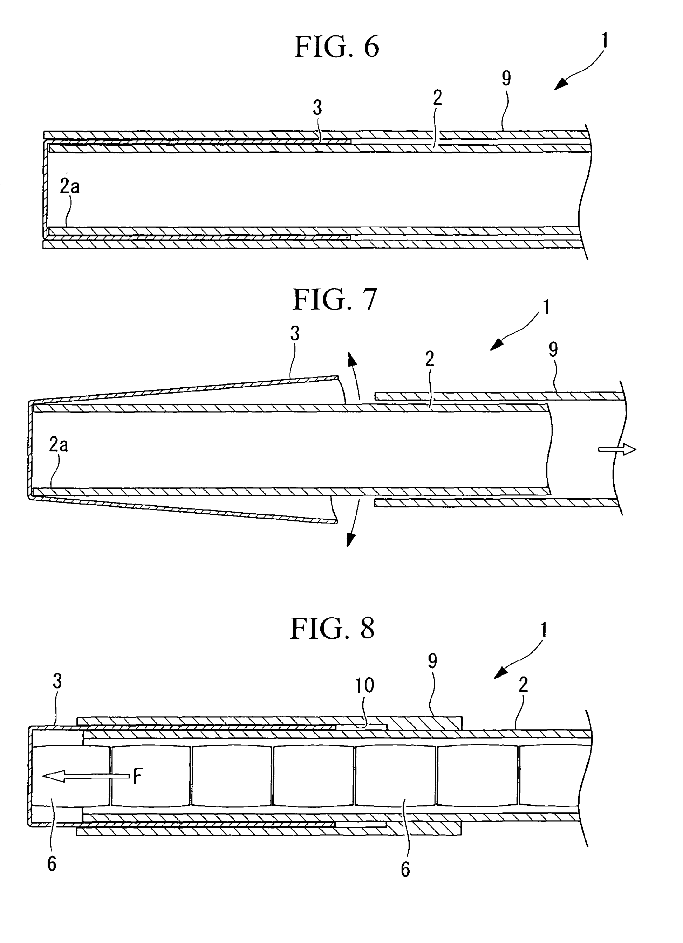

Furthermore, in the present embodiment, the sheet member 3 attached to the front end of the tubular body 2 is exposed during insertion into the introduction hole 5. However, as shown in FIG. 6 and FIG. 7, an outer sheath 9 may be provided that covers the outer surface of the sheet member 3 covering the outer surface of the tubular body 2.
By employing this construction, the sheet member 3 can be sandwiched in the radial direction between the tubular body 2 and the outer sheath 9. Accordingly, the cylindrical shape of the sheet member 3, which has merely been folded, can be easily maintained. Furthermore, because the outer surface of the sheet member 3 is not exposed to the introduction hole 5, problems such as the sheet member 3 adhering to the inside wall of the introduction hole 5 or separating from the tubular body 2 and expanding prematurely as the sheet member 3 travels through the introduction hole 5 can be prevented.

In this case, after the sheet member 3 is introduced into the cavity 8 inside the vertebral bone 7, the outer sheath 9 is withdrawn while the tubular body 2 and the sheet member 3 remain in place, as shown in FIG. 7. This releases the sheet member 3, which is then easily expanded by the compressive force from the filler material 6.

Furthermore, in this case, the tubular body 2 and the outer sheath 9 that sandwich the sheet member 3 in the radial direction are provided as separate components, but an alternative construction may be employed in which, as shown in FIG. 8, the tubular body 2 and the outer sheath 9 are either formed as an integral body, or are secured to each other, thereby forming a circular cylindrical gap 10 into which the sheet member 3 can be inserted in a folded state.
With such a construction, the cylindrical sheet member 3 is pushed out of the gap 10 in the axial direction by the compressive force applied by the filler material 6. At the completion of this process, the sheet member 3 is released from the tubular body 2 and can then expand.

Although in the present embodiment the sheet member 3 is formed from a flexible thin film of a material such as polylactic acid and is expanded by the compressive force imparted by the filler material 6, a sheet member 3 may be used in which at least the frame (not shown) is formed from a material such as a shape memory alloy or shape memory resin that reverts to a remembered shape in response to temperature or other conditions.

By using such a construction, the sheet member 3 remains attached to the front end of the tubular body 2 when introducing the device through the introduction hole 5 from outside the body of the patient, but after the sheet member 3 has been situated in the cavity 8 inside the vertebral bone 7 and a predetermined time has elapsed, the sheet member 3 then expands to a flat shape.
By eliminating the need for a special power source or expansion mechanism, this configuration also provides advantages such as simplifying the construction of, and reducing the size of, the device for vertebral repair 1, as well as improving the ease of use of the device.

Furthermore, although this embodiment was described using polylactic acid as the sheet member 3, a sheet member 3 composed of another biocompatible material such as tetrafluoroethylene may also be used.

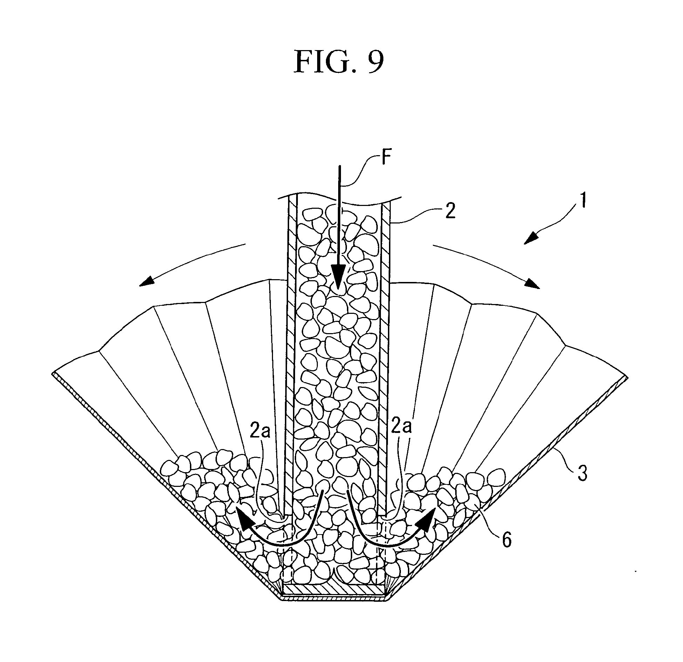

Furthermore, in the present embodiment, the tubular body 2 through which the filler material 6 is injected is a cylindrical tubular body 2 having a front end opening 2a that opens towards the longitudinal direction and is covered by the sheet member 3, but the front end opening 2a of the tubular body 2 may also be provided so as to open in directions transverse to the longitudinal direction of the tubular body 2, as shown in FIG. 9.

In the example shown in FIG. 9, two or more front end openings 2a are provided that open in the radial direction, and when the filler material 6 is a fluid or in a granular or particulate form, the filler material 6 that has traveled through the tubular body 2 changes direction at the front end openings 2a and is discharged in directions transverse to the longitudinal axis.

By employing such a construction, the compressive force applied to the sheet member 3 by the injection of the filler material 6 acts primarily in an outward radial direction. As a result, the compressive force from the filler material 6 causes the folded sheet member 3 attached to the front end of the tubular body 2 to expand in the radial direction.

Such a construction offers an advantage in that the sheet member 3 can be expanded with greater reliability in situations where, for example, the space between the fractured wall surface and the tubular body 2 is empty of contents such as bone marrow and there is little resistance stopping the progress of the sheet member 3.

## Claims

1. A device for vertebral repair used in procedures to repair vertebral fractures, comprising:
a pipe-like tubular body that is inserted into an introduction hole formed in a vertebral bone and can fill the vertebral bone interior with a filler material through an internal through-hole, and
a sheet member attached in a folded state to a front end of the tubular body so as to obstruct an opening at the front end, wherein
the sheet member is attached so as to separate from the tubular body when subjected to a compressive force from the filler material introduced through the through-hole in the tubular body.

2. The device for vertebral repair according to claim 1, wherein the sheet member is folded along an outer surface of the front end of the tubular body, so as to expand under a compressive force from a filler material introduced through a through-hole in the tubular body.

3. The device for vertebral repair according to claim 1, wherein the sheet member is folded along an outer surface of the front end of the tubular body, and is made of a material that expands to a remembered shape when inserted into a vertebral bone.

4. The device for vertebral repair according to either claim 2 or claim 3, comprising an outer sheath that is insertable into the introduction hole and is provided such that a portion of the sheet member along the outer surface of the front end of the tubular body is sandwiched between the outer sheath and the tubular body.

5. The device for vertebral repair according to any one of claim 1 to claim 4, wherein the sheet member is made of a flexible biocompatible material such as polylactic acid or tetrafluoroethylene.

6. The device for vertebral repair according to any one of claim 1 to claim 5, wherein the sheet member is a mesh that is finer than a particle diameter of the filler material.

7. The device for vertebral repair according to claim 2, wherein the opening at the front end of the tubular body is provided so that the filler material that is provided via the through-hole in the tubular body is discharged in directions transverse to a longitudinal direction of the tubular body.
